# FASCICULE DE BREVET EUROPEEN

(11) **EP 0 633 267 B1**
(45) Date de publication et mention de la délivrance du brevet: **26.09.2001**
(21) Numéro de dépôt: 94201642.9
(22) Date de dépôt: 09.06.1994
(51) Int. Cl.: C07K 1/06, C07C 269/00, C07C 271/54

(54) **Procédé de préparation d'amides d'alpha-aminoacide, utilisables en synthèse peptidique**
Verfahren zur Herstellung von alpha-Aminosäureamiden zur Verwendung in der Peptidsynthese
Process for the preparation of alpha-amino acid amide for use in peptide synthesis

(30) Priorité: 18.06.1993 BE 9300622
(43) Date de publication de la demande: 11.01.1995
(73) Titulaire: SOLVAY (Société Anonyme), 1050 Bruxelles (BE)
(72) Inventeur: Callens, Roland, B-9031 Drongen (BE); Blondeel, Georges, B-9300 Aalst (BE)
(74) Mandataire: Jacques, Philippe

(56) Documents cités:
- EP-A- 0 401 817
- EP-A- 0 425 925
- EP-A- 0 477 639
- EP-A- 0 562 659
- DE-A- 3 339 308
- MAKROMOL.CHEM., vol.92, 1966 pages 277 - 286 T.WIELAND ET AL 'Ueber peptidsynthesen,32. Mitt.'
- HELV.CHIM.ACTA, vol.36, no.4, 1953 pages 875 - 886 R.A.BOISSONNAS ET AL 'Etude comparative de la scission de divers groupes de blocage de la fonction alpha-amino des acides aminés'

## Description

La présente invention se rapporte à un nouveau procédé de préparation d'un amide d'α-aminoacide, utilisable en particulier en synthèse peptidique.

Selon une voie classique, un peptide synthétique peut être préparé en solution par addition (condensation) d'un aminoacide, dont le groupement amino est protégé et dont le groupement carboxyle est activé, à une chaîne peptidique ou à un autre aminoacide dans le cas de la préparation d'un dipeptide. Un tel procédé implique cependant un nombre important d'étapes. Ainsi, pour préparer un dipeptide au départ de deux aminoacides simples ne possédant pas de groupements réactifs sur leur chaîne latérale, elle nécessite, préalablement à la réaction de condensation, de protéger le groupement amino du premier aminoacide et le groupement carboxyle du deuxième aminoacide et d'activer le groupement carboxyle du premier aminoacide puis, après la réaction de condensation entre le groupement carboxyle activé du premier aminoacide et le groupement amino libre du deuxième aminoacide, elle implique encore une déprotection du groupement amino du premier aminoacide constituant le dipeptide et du groupement carboxyle du second aminoacide constituant le dipeptide. Un exemple d'un tel procédé est décrit dans "Die Kromoleknlare Chemie", Tome 92, 1966, p. 277-286, dans lequel on obtient un peptide contenant un groupement Nα-3 nitrophénoxycarbonyle. Un autre exemple est décrit dans DE/33 39308, dans lequel on obtient un peptide contenant un groupement Nα-2-hydroxyphényloxycarbonyle.
Le rendement chimique global de la synthèse est bien sûr affecté par le nombre important d'étapes de synthèse, a fortiori lorsqu'un produit chiralement pur est recherché.

L'invention remédie aux inconvénients des procédés classiques de synthèse peptidique en solution en fournissant un nouveau procédé de couplage d'un α-aminoacide avec un composé contenant un groupement amino libre, procédé nécessitant un nombre restreint d'étapes et permettant d'obtenir le produit désiré avec un rendement chimique amélioré, en conservant remarquablement la pureté chirale des structures mises en oeuvre.

En conséquence, l'invention concerne un procédé de préparation d'un amide d'α-aminoacide selon lequel on fait réagir, en milieu basique, un composé contenant un groupement amino libre avec un dérivé de l'aminoacide comportant un groupement N^{α}-aryloxycarbonyle. Ce procédé est particulièrement intéressant en synthèse peptidique.

Par aminoacide, on entend désigner, aux fins de la présente invention, tout composé comprenant au moins un groupement amino et au moins un groupement carboxyle. Par extension, on entend aussi englober ci-après sous le vocable "aminoacide", tout aminoacide tel que défini ci-dessus dont certains autres groupements sont éventuellement liés à des groupements organiques tels que des groupements protecteurs. En particulier, par α-aminoacide, on entend désigner tout aminoacide dans lequel au moins un groupement amino et au moins un groupement carboxyle sont liés au même atome de carbone de la molécule. Il peut s'agir notamment de l'alanine, de la valine, de la leucine, de l'isoleucine, de la proline, de la phénylalanine, du tryptophane, de la méthionine, de la glycine, de la sérine, de la thréonine, de la cystéine, de la tyrosine, de l'asparagine, de la glutamine, de la lysine, de l'arginine, de l'histidine, de l'acide glutamique et de l'acide aspartique.

Par dérivé de l'aminoacide comportant un groupement N^{α}-aryloxycarbonyle, également dénommé N^{α}-aryloxycarbonyl-aminoacide par la suite, on entend désigner tout dérivé d'α-aminoacide dans lequel un groupement aryloxycarbonyle de formule R-O-CO-, avec R symbolisant un groupement aryle, est lié à l'atome d'azote d'un groupement α-amino de l'aminoacide.

Par peptide, on entend désigner, aux fins de la présente invention, tout composé constitué par l'association d'au moins deux aminoacides tels que définis plus haut, dans lequel au moins une liaison entre deux aminoacides est une liaison peptidique normale, c'est-à-dire une liaison amide entre un groupement α-amino d'un aminoacide et un groupement carboxyle d'un autre aminoacide.

Dans le procédé selon l'invention, la mise en oeuvre d'un N^{α}-aryloxycarbonyl-aminoacide est critique. En effet, il est apparu de manière surprenante que le groupement aryloxycarbonyle lié au groupement α-amino de l'aminoacide induit, en présence d'un composé comportant un groupement amino libre, une réaction du groupement carboxyle libre dudit aminoacide avec le groupement amino libre dudit composé et que, de manière concomitante, le groupement amino de l'aminoacide est libéré du groupement aryloxycarbonyle qui y était lié. Le procédé selon l'invention permet donc d'obtenir facilement un amide d'α-aminoacide, exempt de groupement N^{α}-aryloxycarbonyle.

Généralement, le dérivé N^{α}-aryloxycarbonyle de l'aminoacide mis en oeuvre comporte, comme groupement aryloxycarbonyle, un groupement comprenant de 7 à 15 atomes de carbone. Le plus souvent, ce groupement aryloxycarbonyle est un groupement phényloxycarbonyle ou naphtyloxycarbonyle, éventuellement substitué par au moins un groupement choisi parmi les groupements alkyles comprenant de 1 à 4 atomes de carbone, nitro, halogéno et halogénoalkyle. A titre d'exemples de groupements aryloxycarbonyles utilisables dans le procédé de l'invention, on peut citer les groupements phényloxycarbonyle, tolyloxycarbonyle, xylyloxycarbonyle, mésitylyloxycarbonyle, éthylphényloxycarbonyle, diéthylphényloxycarbonyle, propylphényloxycarbonyle, isopropylphényloxycarbonyle, nitrophényloxycarbonyle, dinitrophényloxycarbonyle, chlorophényloxycarbonyle, trichlorométhylphényloxycarbonyle, trifluorométhylphényloxycarbonyle et naphtyloxycarbonyle. De préférence, le groupement aryloxycarbonyle est un groupement phényloxycarbonyle, tolyloxycarbonyle, p-nitrophényloxycarbonyle, 2,4-dinitrophényloxycarbonyle, p-chlorophényloxycarbonyle ou p-trichlorométhylphényloxycarbonyle. De bons résultats ont été obtenus dans le procédé selon l'invention avec le dérivé N^{α}-phényloxycarbonyle de l'aminoacide.

Un dérivé N^{α}-aryloxycarbonyle de n'importe quel α-aminoacide peut être mis en oeuvre dans le procédé selon l'invention.

Le N^{α}-aryloxycarbonyl-aminoacide est un produit facilement accessible et peu coûteux. Il peut être préparé classiquement par une méthode d'acylation similaire à celles utilisées pour fixer sur un groupement amino d'un aminoacide un groupement protecteur de type alkyloxycarbonyle ou aralkyloxycarbonyle. Une méthode classique, connue comme la procédure de Schotten-Baumann consiste à faire réagir, en milieu aqueux, la forme sodique de l'aminoacide avec un agent d'acylation adéquat, tel qu'un chloroformiate d'aryle ou un aryloxycarbonyloxysuccinimide.

Une autre procédure de préparation du N^{α}-aryloxycarbonyl-aminoacide qui a conduit à d'excellents résultats et qui est dès lors préférée, consiste à mettre en oeuvre l'aminoacide sous la forme de son dérivé persilylé. Ce dérivé persilylé peut être obtenu par exemple par un traitement à reflux avec un excès de triméthylcyanosilane, jusqu'à obtention d'une solution homogène. Cette solution est ensuite diluée, par exemple par du chlorure de méthylène, et refroidie à une température inférieure à -10 °C, de préférence inférieure ou égale à -15 °C. La quantité stoechiométrique de chloroformiate d'aryle est alors ajoutée très lentement, puis après quelques minutes de réaction, la solution est concentrée et le N^{α} -aryloxycarbonyl-aminoacide est isolé par la voie la plus appropriée, en fonction de ses propriétés physico-chimiques.

Le procédé permet de préparer des amides primaires, secondaires ou tertiaires, selon la nature du composé contenant le groupement amino libre. Le composé comprenant un groupement amino libre qui réagit avec le N^{α}-aryloxycarbonyl-aminoacide dans le procédé selon l'invention est tout composé de formule générale R1R2NH dans laquelle R1 et R2 représentent indépendamment l'un de l'autre, des atomes d'hydrogène, des radicaux alkyles, cycloalkyles ou aralkyles ou dans laquelle R1 et R2 forment ensemble un radical alicyclique. Dans ce composé, les radicaux alkyles, cycloalkyles, aralkyles ou alicycliques peuvent être substitués par un ou plusieurs groupements fonctionnels comprenant au moins un atome d'oxygène, de soufre ou d'azote, tels que notamment un groupement carboxyle, hydroxyle, mercapto, indolyle ou imidazolyle. Des composés utilisables dans le procédé selon l'invention sont, par exemple, l'ammoniac, les amines primaires ou secondaires, les aminoacides et les peptides. Le procédé selon l'invention est particulièrement intéressant lorsque le composé comprenant un groupement amino libre est un aminoacide. Il constitue dans ce cas un procédé très aisé de synthèse de dipeptides.

Le procédé selon l'invention est effectué pans un milieu basique.

Le procédé selon l'invention est effectué généralement dans un milieu liquide aqueux comprenant, de préférence, un solvant organique miscible à l'eau, milieu dans lequel le N^{α}-aryloxycarbonyl-aminoacide et le composé comprenant un groupement amino libre sont au moins en partie solubles. Des solvants organiques qui conviennent dans le procédé selon l'invention sont les alcools inférieurs tels que notamment le méthanol, l'éthanol et l'isopropanol, le tétrahydrofurane et le diméthoxyéthane. De bons résultats ont été obtenus dans un milieu eau-éthanol. Le rapport pondéral eau / solvant organique peut varier dans de larges mesures. En général, il est au moins égal à 0,1 et ne dépasse pas 10.

La basicité du milieu peut être obtenue par ajout d'un composé basique dans le milieu, par exemple par ajout d'une base inorganique telle que LiOH, NaOH ou KOH ou par ajout d'une base organique inerte dans les conditions de réaction, telle qu'une amine tertiaire. De bons résultats ont été obtenus en présence de triéthylamine. Lorsque le composé contenant un groupement amino libre est un aminoacide ou un peptide comportant des fonctions carboxyles libres, le composé basique doit être mis en oeuvre en quantité suffisante pour neutraliser les fonctions carboxyles. Lorsque le composé contenant un groupement amino libre présente un caractère basique suffisant, la basicité du milieu peut éventuellement être obtenue en travaillant avec un excès de ce composé par rapport à la quantité stoechiométrique nécessaire pour réagir avec le N^{α}-aryloxycarbonyl-aminoacide.

Le procédé selon l'invention peut être mis en oeuvre dans une large gamme de concentration des réactifs dans le milieu liquide. En général, le N^{α}-aryloxycarbonyl-aminoacide est mis en oeuvre à une concentration de 0,05 à 5 mole/l, de préférence de 0,1 à 1 mole/l.

Le rapport molaire entre le composé contenant un groupement amino libre et le N^{α}-aryloxycarbonyl-aminoacide est généralement au moins égal à 1. De préférence, ce rapport est au moins égal à 1,1. En principe, il n'y a pas de limite supérieure à ce rapport. En pratique, il est généralement inutile de travailler avec un rapport molaire entre le composé contenant un groupement amino libre et le N^{α} -aryloxycarbonyl-aminoacide supérieur à 100. Le plus souvent, le rapport molaire ne dépasse pas 10. Lorsque le composé contenant un groupement amino libre est un aminoacide ou un peptide, le rapport molaire ne dépasse de préférence pas 5.

La réaction peut être effectuée de la température ambiante jusqu'à la température d'ébullition du solvant organique. Elle est avantageusement effectuée de 30 à 80 °C. Une température de 40 à 60 °C est tout particulièrement préférée.

Dans ces conditions, le temps de réaction est généralement inférieur à 10 heures. Le plus souvent, la réaction est complète après une durée de 1 à 4 heures.

Le procédé selon l'invention apparaît particulièrement avantageux par rapport aux procédés connus de préparation d'amides d'α-aminoacides. Le dérivé N^{α}-aryloxycarbonyle de l'aminoacide mis en oeuvre dans le procédé selon l'invention peut être préparé facilement et à peu de frais au départ de l'aminoacide. Il peut être aisément isolé sous forme pure. Il est stable et peut être conservé longtemps. Le procédé selon l'invention est particulièrement intéressant en synthèse peptidique, c'est-à-dire lorsque le composé comprenant un groupement amino libre est un aminoacide ou un peptide. Il constitue, dans ce cas, un procédé de synthèse peptidique particulièrement simple, performant et très respectueux de la chiralité des composés mis en oeuvre. Dans le procédé selon l'invention, le groupement aryloxycarbonyle joue à la fois le rôle de groupement protecteur du groupement α-amino et d'activateur de la réaction de condensation du groupement carboxyle avec un groupement amino libre d'un autre composé. De plus, lorsque le dérivé N^{α}-aryloxycarbonyle de l'aminoacide réagit avec le composé contenant un groupement amino libre, le groupement amino de l'aminoacide est simultanément libéré du groupement aryloxycarbonyle qui y était lié. En outre, le départ du groupement aryloxycarbonyle ne génère dans le milieu que des sous-produits relativement inoffensifs qui ne perturbent pas la synthèse. Par exemple, lorsqu'il s'agit du groupement phényloxycarbonyle, seuls du phénol et du dioxyde de carbone sont générés. Dès lors, lorsque les composés mis en oeuvre comportent des groupements très labiles, tels que certains groupements protecteurs, les sous-produits générés dans le milieu ne provoquent aucune dégradation de ces composés. Par rapport aux voies de synthèse classiques, un peptide synthétique peut être préparé par le procédé selon l'invention de manière nettement plus simple. Ainsi, pour préparer un dipeptide au départ de deux aminoacides simples ne possédant pas de fonctions réactives sur leur chaîne latérale, le procédé selon l'invention ne demande que deux étapes simples, à savoir, dans une première étape, de fixer sur le groupement amino du premier aminoacide un groupement aryloxycarbonyle et, dans une deuxième étape, de faire réagir selon le procédé de l'invention le N^{α}-aryloxycarbonyl-aminoacide obtenu avec le deuxième aminoacide . Le recours à une étape d'activation du groupement carboxyle du premier aminoacide ou à un agent de couplage est superflu, de même que toute étape de déprotection. Le procédé selon l'invention est dès lors tout particulièrement indiqué pour la préparation de dipeptides.

Les représentations symboliques des aminoacides et des peptides adoptées dans la description et les exemples suivent les recommandations IUPAC de nomenclature, généralement adoptées et décrites par exemple dans "Nomenclature and Symbolism for Amino Acids and Peptides, Recommendations 1983", Eur. J. Biochem. (1984), 138, p.9-37. Sauf stipulation contraire, tous les aminoacides décrits sont des (L)-aminoacides.

Les exemples suivants illustrent l'invention.

Les différents produits et intermédiaires de synthèse rapportés dans les exemples ont été caractérisés par différentes méthodes analytiques, mises en oeuvre dans les conditions suivantes :
- rotation optique (α) : mesurée à 589 nm à 25 °C
- Chromatographie en couche mince (CCM) :
   . Plaques de silicagel MERCK 60F-254
   . éluants : Rf(1) Acétate d'éthyle:n-butanol:acide acétique:eau 1:1:1:1
      Rf(2) Acétonitrile:chloroforme:acide acétique:eau 5:2:2:1
- Chromatographie HPLC :
   . Colonne C-18 Vydac 5µm
   . Elution : gradient de 98 % A + 2 % B jusqu'à 25 A + 75 B en 49 minutes (A = 0,1 % acide trifluoroacétique dans eau ; B = 0,1 % acide trifluoroacétique dans acétonitrile)
   . Débit = 2 ml/min
   . Détection : UV 220 nm.
- Résonance magnétique nucléaire (RMN) :
   . Appareil Brüker AMX 500 MHz
   . Shift donnés en ppm
   . Allures des résonances : m=multiplet, s=singulet, d=doublet, t=triplet, q=quadruplet, quint=quintuplet, o=octuplet.

### Exemple 1 : synthèse de tryptophyl-alanine et de tryptophyl-(D)-alanine

2,922 g (9 mmol) de N^{α}-phényloxycarbonyl-tryptophane ont été dissous dans 20 ml de méthanol. 3,204 g (36 mmol) de (D,L)-alanine ont été dissous dans 20 ml d'eau et convertis en sel de triéthylamine par addition de 3,643 g de l'amine. Les deux solutions ont été mélangées, puis chauffées sous léger reflux, jusqu'à disparition du N^{α}-phényloxycarbonyl-tryptophane. Le méthanol a été chassé par évaporation sous pression réduite et le résidu neutralisé avec 4,9 g (36 mmol) de KHSO4 dans 30 ml d'eau. Après extraction par de l'acétate d'éthyle de la majeure partie du phénol libéré lors de la réaction, la phase aqueuse a été diluée à 250 ml puis purifiée par chromatographie préparative (phase inverse C18 DeltaPak). Les fractions pures ont été rassemblées puis lyophilisées pour donner 1,12 g de tryptophyl-alanine (Trp-Ala) et 0,66 g de tryptophyl-(D)-alanine (Trp-(D)-Ala) optiquement purs. Les produits obtenus présentent les propriétés physico-chimiques suivantes :
Trp-Ala
α = + 17,9 (c = 1, 1% acide acétique)
PF = 104 °C
CCM : Rf(1) = 0,63
HPLC tR: 8,44 min
RMN (¹H) dans le DMS0-d6
10,92 (1H s) NH indole 8,45 (1H s large) NH Ala
7,63 (1H d) H4 indole 7,32 (1H d) H7 indole
7,20 (1H s) H2 indole 7,05 (1H t) H6 indole
6,97 (1H t) H5 indole 4,16 (1H q large) Hα Ala
3,76 (1H dd) Hα Trp 3,19 (1H dd) HβA Trp
2,90 (1H dd) HβB Trp 1,25 (1H t) CH3 Ala
Trp-(D)-Ala
α = + 79,1 (c = 1, 1% acide acétique)
PF = 107 °C
CCM: Rf(1) = 0,59
HPLC tR: 11,67 min
RMN (¹H) dans le DMS0-d6
10,88 (1H s) NH indole 8,20 (1H s large) NH Ala
7,58 (1H d) H4 indole 7,32 (1H d) H7 indole
7,16 (1H s) H2 indole 7,05 (1H t) H6 indole
6,97 (1H t) H5 indole 4,05 (1H q large) Hα Ala
3,73 (1H dd) Hα Trp 3,12 (1H dd) HβA Trp
2,88 (1H dd) HβB Trp 1,12 (1H t) CH3 Ala

### Exemple 2 : Synthèse de (ε-tert.-butyloxycarbonyl)-lysyl-(ε-tert.-butyloxycarbonyl)-lysine ((Boc)Lys-(Boc)Lys)

1,46 g (4 mmol) de N^{ε}-tert.-butyloxycarbonyl-N^{α}-phényloxycarbonyl-lysine ont été dissous dans 16 ml d'éthanol. Y ont été rajoutés 1,13 g (4,6 mmol) de N^{ε}-tert.-butyloxycarbonyl-Lysine, 1,91 ml (13,8 mmol) de triéthylamine et 4 ml d'eau. La solution a été chauffée sous léger reflux (± 80°C) pendant 5 h, puis refroidie à température ambiante et neutralisée par 12,2 ml d'acide chlorhydrique 1N. L'éthanol a été éliminé par évaporation sous vide. Le résidu (± 15 g) a été mis dans le réfrigérateur pendant 3 h. Le précipité formé a été isolé par filtration, puis lavé avec 7 ml d'eau froide et 25 ml d'éther sulfurique. Après séchage, 1,42 g de (Boc)Lys-(Boc)Lys ont été obtenus, ce qui correspond à un rendement de 75 %. Un échantillon recristallisé dans un mélange méthanol:eau avait les propriétés physico-chimiques suivantes :
α = +19,2 (c = 1, méthanol)
PF = 180-189 °C
CCM : Rf(1) = 0,85
HPLC tR= 20,79 min
RMN (¹H) dans le CD₃0D
4,26 (1H dd) Hα Lys2 3,86 (1H t) HαLysl
3,04 (4H quint) Hε's Lys1+2 1,95 à 1,80 (3H m) Hβ's Lys
1,73 (1H m) Hβ Lys 1,50 à 1,40 (26H m) Hγ+δ+t. Bu

Cet exemple illustre l'intérêt tout particulier du procédé selon l'invention pour préparer des homodipeptides, lesquels peuvent être obtenus avec un rapport molaire entre le composé contenant un groupement amino libre et le N^{α}-aryloxycarbonyl-aminoacide quasiment stoechiométrique.

### Exemple 3 : Synthèse de S-(trityl)-cystéinamide ((Trt)Cys-NH2)

48,5 g (100 mmol) de N^{α}-phényloxycarbonyl-S-trityl-cystéine ont été dissous dans 200 ml de tétrahydrofurane. Y ont été rajoutés 135 ml d'ammoniaque concentré à 25 %. (± 1,75 mol). Cette solution a été chauffée pendant 5 h à 50 °C, sous agitation. Après refroidissement, le mélange réactionnel a été dilué avec 400 ml de méthyl-tert.-butyl éther. La phase aqueuse obtenue a été rejetée et la phase organique lavée 3 fois par 200 ml d'une solution aqueuse contenant 5 % de NaCl. Elle a ensuite été traitée avec 50 ml d'eau, puis le S-(trityl)-cystéinamide a été précipité sous forme d'hémisulfate par addition de 13,6 g de KHSO4 dissous dans 70 ml d'eau. Il a été isolé par filtration, lavé avec 150 ml d'eau froide puis séché. 29 g de produit ont été obtenus (rendement : 71 %). Le produit présente les propriétés physico-chimiques suivantes :
= +18 (c = 1, méthanol)
CCM : Rf(2) = 0,80
HPLC : tR = 25,3 min
RMN (¹H) dans le CD3OD (+ une goutte d'acide trifluoroacétique)
7,42 (6H d) 7,32 (6H t)
7,26 (3H d) o. m. p. trityl
3,53 (1H t) Hα 2,67 (2H o) Hβ's

Cet exemple démontre que le procédé selon l'invention peut être utilisé avec succès pour préparer l'amide d'un aminoacide portant, en bout de chaîne latérale, un groupement protecteur très labile.

## Revendications

1. Procédé de préparation d'un amide d'α-aminoacide exempt de groupement N^{α}-aryloxycarbonyle selon lequel on fait réagir, en milieu basique, un composé contenant un groupement amino libre avec un dérivé de l'aminoacide comportant un groupement N^{α}-aryloxycarbonyle.

2. Procédé selon la revendication 1, **caractérisé en ce que** le dérivé N^{α}-aryloxycarbonyle de l'aminoacide mis en oeuvre comporte, comme groupement aryloxycarbonyle, un groupement comprenant de 7 à 15 atomes de carbone.

3. Procédé selon la revendication 2, **caractérisé en ce que** le groupement aryloxycarbonyle est un groupement phényloxycarbonyle ou naphtyloxycarbonyle, éventuellement substitué par au moins un groupement choisi parmi les groupements alkyles comprenant de 1 à 4 atomes de carbone, nitro, halogéno et halogénoalkyle.

4. Procédé selon la revendication 3, **caractérisé en ce que** le groupement aryloxycarbonyle est un groupement phényloxycarbonyle, tolyloxycarbonyle, p-nitrophényloxycarbonyle, 2,4-dinitrophényloxycarbonyle, p-chlorophényloxycarbonyle ou p-trichlorométhylphényloxycarbonyle.

5. Procédé selon la revendication 4, **caractérisé en ce que** le groupement aryloxycarbonyle est le groupement phényloxycarbonyle.

6. Procédé selon une quelconque des revendications 1 à 5, **caractérisé en ce que** le composé comprenant un groupement amino libre est choisi parmi l'ammoniac, les amines primaires ou secondaires, les aminoacides et les peptides.

7. Procédé selon la revendication 6, **caractérisé en ce que** le composé comprenant un groupement amino libre est un aminoacide.

8. Procédé selon une quelconque des revendications 1 à 7, **caractérisé en ce qu'**il est effectué dans un milieu liquide aqueux comprenant un solvant organique miscible à l'eau.

9. Procédé selon une quelconque des revendications 1 à 8, **caractérisé en ce que** le N^{α}-aryloxycarbonyl-aminoacide est mis en oeuvre à une concentration de 0,05 à 5 mole/l.

10. Procédé selon une quelconque des revendications 1 à 9, **caractérisé en ce que** le rapport molaire entre le composé contenant un groupement amino libre et le N^{α}-aryloxycarbonyl-aminoacide est au moins égal à 1.

## Patentansprüche

1. Verfahren zur Herstellung eines α-Aminosäureamids, das frei von der N^{α}-Aryloxycarbonylgruppe ist, gemäß dem man in basischem Medium eine Verbindung, die eine freie Aminogruppe enthält, mit einem Derivat der Aminosäure, das eine N^{α} -Aryloxycarbonylgruppe umfasst, umsetzt.

2. Verfahren gemäß Anspruch 1, **dadurch gekennzeichnet, dass** das N^{α}-Aryloxycarbonylderivat der Aminosäure, das verwendet wird, als Aryloxycarbonylgruppe eine Gruppe mit 7 bis 15 Kohlenstoffatomen umfasst.

3. Verfahren gemäß Anspruch 2, **dadurch gekennzeichnet, dass** die Aryloxycarbonylgruppe eine Phenyloxycarbonyl- oder Naphthyloxycarbonylgruppe ist, die gegebenenfalls mit wenigstens einer Gruppe substituiert ist, die unter den Alkylgruppen mit 1 bis 4 Kchlenstoffatomen, Nitro, Halogen und Halogenalkyl ausgewählt ist.

4. Verfahren gemäß Anspruch 3, **dadurch gekennzeichnet, dass** die Aryloxycarbonylgruppe eine Phenyloxycarbonyl-, Tolyloxycarbonyl-, p-Nitrophenyloxycarbonyl-, 2,4-Dinitrcphenyloxycarbonyl-, p-Chlorphenyloxycarbonyl- oder p-Trichlormethylphenyloxycarbonylgruppe ist.

5. Verfahren gemäß Anspruch 4, **dadurch gekennzeichnet, dass** die Aryloxycarbonylgruppe die Phenyloxycarbonylgruppe ist.

6. Verfahren gemäß einem der Ansprüche 1 bis 5, **dadurch gekennzeichnet, dass** die Verbindung, die eine freie Aminogruppe umfasst, unter Ammoniak, den primären oder sekundären Aminen, den Aminosäuren und den Peptiden ausgewählt ist.

7. Verfahren gemäß Anspruch 6, **dadurch gekennzeichnet, dass** die Verbindung, die eine freie Aminogruppe umfasst, eine Aminosäure ist.

8. Verfahren gemäß einem der Ansprüche 1 bis 7, **dadurch gekennzeichnet, dass** es in einem wässrigen flüssigen Medium, das ein mit Wasser mischbares organisches Lösungsmittel umfasst, ausgeführt wird,

9. Verfahren gemäß einem der Ansprüche 1 bis 8, **dadurch gekennzeichnet, dass** die N^{α}-Aryloxycarbonyl-Aminosäure in einer Konzentration von 0,05 bis 5 Mol/l verwendet wird.

10. Verfahren gemäß einem der Ansprüche 1 bis 9, **dadurch gekennzeichnet, dass** das Molverhältnis zwischen der Verbindung, die eine freie Aminogruppe umfasst, und der N^{α}-Aryloxycarbonyl-Aminosäure wenigstens gleich 1 ist.

## Claims

1. Process for the preparation of an α-amino acid amide free of an N^{α}-aryloxycarbonyl group, according to which a compound containing a free amino group is reacted, in basic medium, with a derivative of the amino acid containing an N^{α}-aryloxycarbonyl group.

2. Process according to Claim 1, **characterized in that** the N^{α} -aryloxycarbonyl derivative of the amino acid used includes a group containing 7 to 15 carbon atoms as aryloxycarbonyl group.

3. Process according to Claim 2, **characterized in that** the aryloxycarbonyl group is a phenyloxycarbonyl or naphthyloxycarbonyl group, optionally substituted with at least one group chosen from alkyl groups containing 1 to 4 carbon atoms and nitro, halo and haloalkyl groups.

4. Process according to Claim 3, **characterized in that** the aryloxycarbonyl group is a phenyloxycarbonyl, tolyloxycarbonyl, p-nitrophenyloxycarbonyl, 2,4-dinitrophenyloxycarbonyl, p-chlorophenyloxycarbonyl or p-trichloromethylphenyloxycarbonyl group.

5. Process according to Claim 4, **characterized in that** the aryloxycarbonyl group is the phenyloxycarbonyl group.

6. Process according to any one of Claims 1 to 5, **characterized in that** the compound containing a free amino group is chosen from ammonia, primary or secondary amines, amino acids and peptides.

7. Process according to Claim 6, **characterized in that** the compound containing a free amino group is an amino acid.

8. Process according to any one of Claims 1 to 7, **characterized in that** it is carried out in an aqueous liquid medium containing a water-miscible organic solvent.

9. Process according to any one of Claims 1 to 8, **characterized in that** the N^{α}-aryloxycarbonylamino acid is used at a concentration of 0.05 to 5 mol/l.

10. Process according to any one of Claims 1 to 9, **characterized in that** the molar ratio between the compound containing a free amino group and the N^{α} -aryloxycarbonylamino acid is at least equal to 1.
